Europäisches Patentamt

European Patent Office

Office européen des brevets

(11) Publication number: **0 350 052**
A2

(12) # EUROPEAN PATENT APPLICATION

(21) Application number: 89112399.4

(22) Date of filing: 06.07.89

(51) Int. Cl.⁴: **A01K 67/02** , **C12P 21/00** , **C12N 15/85**

(30) Priority: 06.07.88 JP 166992/88
10.11.88 JP 284367/88
10.11.88 JP 284641/88

(43) Date of publication of application:
10.01.90 Bulletin 90/02

(34) Designated Contracting States:
AT BE CH DE ES FR GB GR IT LI NL SE

(71) Applicant: DAIICHI PHARMACEUTICAL., LTD.
14-10, Nihonbashi 3-chome Chuo-ku
Tokyo(JP)

(72) Inventor: Ariga, Hiroyoshi
404-33, Nakanoshima 1-jo 7-chome
Toyohira-ku
Sapporo-shi Hokkaido(JP)
Inventor: Sato, Yoshinari c/o Daiichi
Pharmaceutical Ltd.
Research Institute 16-13, Kitakasai 1-chome
Edogawa-ku Tokyo(JP)
Inventor: Shibamura, Seiichi c/o Daiichi
Pharmaceutical Ltd.
Research Institute 16-13, Kitakasai 1-chome
Edogawa-ku Tokyo(JP)
Inventor: Furusawa, Mitsuru c/o Daiichi
Pharmaceutical Ltd.
Research Institute 16-13, Kitakasai 1-chome
Edogawa-ku Tokyo(JP)

(74) Representative: Kinzebach, Werner, Dr. et al
Patentanwälte Reitstötter, Kinzebach und
Partner Sternwartstrasse 4 Postfach 86 06 49
D-8000 München 86(DE)

(54) Transgenic animals transformed with autonomously replicating sequence-containing plasmid.

(57) A transgenic animal carrying in the reproductive cells and somatic cells thereof a plasmid containing a mammalian cell-derived autonomously replicating sequence DNA, a promoter sequence and a peptide gene, said plasmid being maintained stably in the episome state and replicating extrachromosomally.

# TRANSGENIC ANIMALS TRANSFORMED WITH AUTONOMOUSLY REPLICATING SEQUENCE-CONTAINING PLASMID

## FIELD OF THE INVENTION

This invention relates to transgenic animals carrying an autonomously replicating sequence-containing plasmid introduced thereinto, a method of producing said animals and a method of producing peptides using said animals. This invention also relates to autonomously replicating sequence DNA.

## BACKGROUND OF THE INVENTION

Transgenic animals transformed or transfected by introduction of a recombinant DNA into animal germs or embryos are known. Thus, for instance, mention may be made of transgenic mice carrying the human globin gene (References 1 and 2), transgenic mice carrying the rabbit globin gene (References 3 and 4), transgenic mice carrying the chicken transferrin gene (Reference 5), transgenic mice carrying a functionally rearranged immunoglobulin gene (Reference 6), transgenic mice carrying the rat growth hormone gene fused to the mouse metallothionein (MMT) promoter (Reference 7), transgenic mice carrying the thymidine kinase gene joined to the MMT promoter (Reference 8), transgenic mice carrying the human growth hormone gene fused to the MMT promoter (Reference 9) and nonhuman transgenic animals carrying an activated oncogene sequence (Reference 10). Transgenic mammals carrying a foreign gene joined to the milk protein promoter (Reference 11) are also known.

The use of such transgenic animals makes it possible to produce desired peptides in markedly larger quantities as compared with the prior art methods of producing desired peptides through recombinant DNA expression in microorganisms or animal cells. Once appropriate transgenic animals have been obtained, their offspring can also be used in efficient, large-quantity production of desired peptides.

No animal cell-derived plasmid has been found as a replication unit capable of replicating in animal cells. Therefore, parts of viral genomes are used in constructing plasmid vectors to be harbored in animal cells. Consequently, the range of host animals usable for introduction of vectors thereinto is limited depending on the virus employed.

Plasmids introduced into animal cells either as such or after incorporation into host chromosomes will not autonomously replicate independently. This is because even when the conventional plasmid vectors contain a viral Ori (replication origin, or initiation site for replication), this Ori cannot function in animal cells where proteins and other substances required for viral DNA replication are absent. Therefore, plasmid vectors, when incorporated into host chromosomes, are replicated during chromosome replication as portions of the chromosomes. As a result, the copy number of a gene introduced is inevitably small, so that the expression of the gene introduced, namely the production of the desired peptide, cannnot be expected to reach a satisfactory level. Furthermore, the site of insertion of a foreign gene into a host chromosome cannot be chosen. In case of insertion at an abnormal site, a gene abnormality may disadvantageously result in host animals.

The replication origin in the autonomous replication of an animal chromosome is present in an autonomously replicating sequence (ARS). This ARS is a DNA sequence autonomously replicating and amplifying in eukaryotic cells. While some sequences capable of functioning in yeasts have been reported (Reference 12), none have been found in higher mammals.

## Summary of the Invention

The present inventors have discovered for the first time a mouse DNA-derived ARS (pARS65) capable of functioning in mouse cells (Reference 13) and have reported that this ARS can function also in heterologous cells, such as rat and human cells (Reference 14). The present inventors have further established that a c-myc protein-binding DNA sequence from the human promyelocytic leukemia cell line HL-60 is an ARS (pHLmyc) and that an ARS (pmyc(H-P)) is present in the HindIII-PstI region of the human c-myc gene upstream region and this ARS has homology to a mouse ARS (pARS65 and a human ARS (pHLmyc) (Reference 15).

The present inventors have now found that when an ARS-containing plasmid (cf. Reference 15) is introduced into animal embryonic cells, this plasmid autonomously replicates and is stably maintained episomally, namely even though it is not incorporated into the chromosomes. The present inventors further investigated the HindIII-PstI region of the human c-myc gene and determined an ARS DNA sequence. The present invention, which has been completed based on these findings, provides transgenic animals carrying a plasmid which is not a viral vector, a method of producing said animals and a method of utilizing said animals and a novel ARS.

Accordingly, a primary object of the invention is to provide a transgenic animal which allows large quantity replication of a plasmid introduced and large quantity expression of a desired peptide and in which no damage to the host chromosomal DNA occurs.

A second object of the invention is to provide a method of producing such a transgenic animal.

A third object of the invention is to provide a method of producing a desired peptide using said transgenic animal.

A fourth object of the invention is to provide a novel autonomously replicating sequence.

The first object of the invention is accomplished by providing a transgenic animal harboring in the reproducitive cells and somatic cells thereof a plasmid containing a mammalian cell-derived autonomously replicationg sequence DNA, a promoter sequence and a peptide gene.

The second object of the invention is accomplished by providing a method of producing a transgenic animal which comprises introducing into an embryonic cell a plasmid containing a mammalian cell-derived autonomously replicating sequence DNA, a promoter sequence and a peptide gene.

The third object of the invention is accomplished by providing a method of peptide production which comprises causing said peptide gene to be expressed in somatic cells of said trasgenic animal.

The fourth object of the present invention is accomplished by providing the following base sequence.

$$T \quad C \quad T \quad C \quad T \quad T \quad A \quad T \quad G \quad C \quad G \quad G \quad T \quad T \quad G \quad A \quad A \quad T \quad A \quad G \quad T$$

## BRIEF DESCRIPTION OF THE DRAWINGS

Fig. 1 is the restriction enzyme cleavage map of the human c-myc gene upstream region. In the figure, the symbol □ indicates a noncoding exon, the symbol ■ represents a coding exon and the symbols $P_1$ and $P_2$ each represents a promoter.

Fig. 2 shows Southern blot patterns of tail DNA samples derived from transgenic $F_2$ mice (15 males and 13 females) and

Fig. 3 shows Southern blot patterns of tail DNA samples derived from mice obtained by $F_0$ (male) x $F_1$ (female) backcrossing. In each figure, the migration start line is at the top. The arrow indicates the position of pmyc(H-P). The two bands occurring above the position of the arrow correspond to the dimer and trimer of pmyc(H-P), respectively.

Fig. 4 shows a Southern blot pattern which indicates the distribution of pmyc(H-P) in a $F_2$ mouse.

Fig. 5 and Fig. 6 each shows the estimated secondary structure of the ARS of pHLmyc.

Fig. 7 shows the estimated secondary structure of the ARS of pmyc(H-P).

Fig. 8 comparatively shows the ARS of pmyc(H-P), mouse ARS (pARS65) and human ARS (pHLmyc). In the figure, the asterisks indicate the coincidental portions and the underlined portions are the stem portions of stem/loop structures presumably essential to ARS.

Fig. 9 shows a Southern blot pattern which indicates the result of gel shift assay of a synthetic 21-bp DNA. In the figure, the right end is the migration starting line.

Fig. 10 shows a blot pattern which indicates the result of gel shift assay of the synthetic 21-bp DNA. In the figure, the migration starting line is on the top. The DNA sequence shown a top in the figure is the sequence of a DNA derived from the synthetic 21-bp ARS (underlined) by adding a BamHI site to each end and inserting into pSVPCAT. The DNA was used in the gel shift assay.

Fig. 11 shows a thin layer chromatogram which indicates the result of CAT assay of the synthetic 21-bp DNA. In the figure, the arrow indicates the extent of migration of acetylated chloramphenicol.

## DETAILED DESCRIPTION OF THE INVENTION

The ARS (autonomously replicating sequence) can be obtained as a DNA sequence region having specific affinity for DNA-binding proteins.

These DNA-binding proteins are nonhistone proteins occurring in cell nuclei and, in the DNA-bound form, control and regulate the functioning and expression of DNA. For example, the c-myc protein (cf. Reference 16), the v-myc protein (cf. Reference 17), the N-myc protein (cf. Reference 18), the c-myb protein (cf. Reference 18), the v-myb protein (cf. Reference 18), the c-fos protein (cf. Reference 18), the v-fos protein (cf. Reference 18), p53 (cf. Reference 18), the RB gene product (cf. Reference 19) and like myc and myb proteins are known as such proteins. These proteins may be available in cell nuclear extract as described in the above references. The isolation of the proteins from the cell nuclear extract can be carried out by affinity chromatography which comprises applying a protein-containing buffer solution to a column in which a DNA-binding resin is packed and eluting the protein which is bound to the DNA.

Cells which serve as the source of ARS are not limited to any particular species. However, cells producing DNA-binding proteins in large amounts are preferably used as the ARS source. Examples of cells capable of producing the proteins, include, for example, human HL-60 cells, human IML cells, human Raji cells and mouse FM3A cells. The ARS is not limited to mouse- or human-derived ARS but may also include those derived from mammals such as rats, guinea pigs, cattle, sheep, goats, rabbits and monkeys.

The ARS can be obtained from the animal cell DNA by the method described in European Patent Application EP0254315A2, the disclosure of which is incorporated herein by reference. Thus, chromosomal DNA isolated from mammalian cells is digested with an appropriate restriction enzyme or enzymes to produce DNA fragments appropriate in length (generally 1,000 to 10,000 bp). These are incubated with a DNA-binding protein to give DNA-protein conjugates, which are then precipitated as immune complexes by addition of an anti-DNA-binding protein antibody as a first antibody and protein A as a second antibody. Extraction of this precipitate with phenol gives a desired ARS-containing DNA fragment. After confirmation that this fragment has ARS activity, the DNA fragment, either as such or after modification in length to obtain a fragment having an appropriate length as necessary, can be used to construct vectors for animal cells.

The antibody to the DNA-binding protein can be produced by a general manner such as by immunizing an animal with the protein but it is convenient to obtain from commercial sources such as Oncor Inc., U.S.A. and etc. For example, the third exon of N-myc gene (the specific moiety of N-myc) is expressed in E. coli to obtain N-myc protein then a mouse is immuninized with the protein by a general method. Anti-serum is collected from the mouse and the IgG fraction thereof is used as the anti-N-myc protein antibody (Reference 20).

The second antibody can also be produced by a method known per se, and protein A are provided as a form of cell suspension of Staphylococcus aureus (P-7155 (Sigma)) or a combined form with some solid (Protein A-Sepharose CL-4B (Pharmacia) or Protein A-Agarose FPA-1 (Cosmo-Bio Ltd.).

Separation of the DNA from the complex (e.g., DNA-binding protein-antibody-protein A) can be carried out in a conventional manner, for example, by treatment with a reagent such as 2-mercaptoethanol or a proteolytic enzyme (Reference 21) such as Proteinase K (Proteinase K P0390 (Sigma), Proteinase K No. 24568 (Merck)), however, 2-mercaptoethanol is preferable since the proteolytic enzyme may sometimes degradate DNA chain.

The ARS-containing DNA fragment can be utilized either in the form of ARS itself or of an ARS-containing DNA fragment having an appropriate length, as desired.

The size of possible ARS DNA is considered in about one hundred base pairs, however, it is also supposed that sometimes the ARS DNA is partially overlapped with an enhancer DNA. So, use can be made of a DNA having several hundreds base pairs as ARS-containing DNA fragment.

For example, the ARS contained in such plasmids as pHLmyc, pmyc(H-P), pmyc(H-K), pIMR-N, pRJ-53 and pARS65 (Reference 15) usable as such ARS.

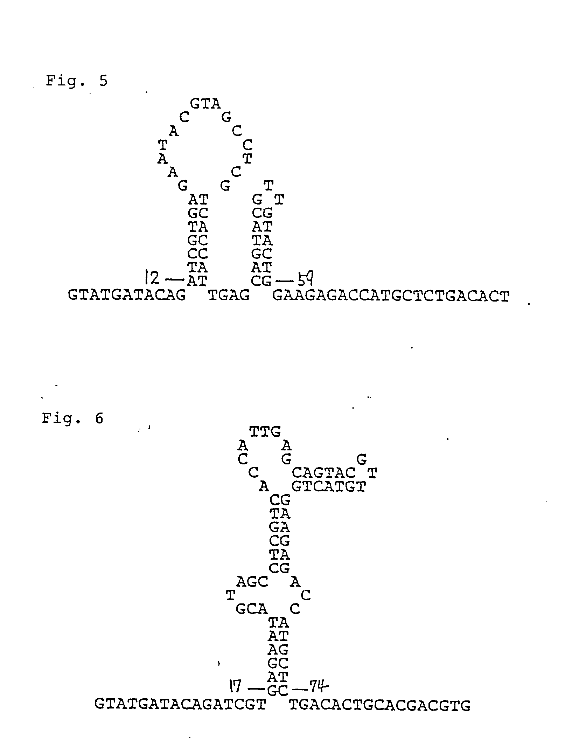

pHLmyc is a human ARS-containing plasmid constructed by isolation of a c-myc protein-binding fragment from among HindIII-HindIII fragments of the human HL-60 cell chromosomal DNA and insertion of the fragment into pUC19 (Pharmacia), followed by rearrangement in bacterial cells. It is grown in E. coli K12 C600 ARS-2 (deposited at the Fermentation Research Institute under the deposit number FERM BP-1444 in accordance with the Budapest Treaty). Its secondary structure anticipated from the DNA sequence of its 99-base-long polylinker HindIII site is the stem/loop structure shown in Fig. 5 or Fig. 6. The bases 12 to 59 or the bases 17 to 74 are considered as an important ARS base sequence.

pmyc(H-K) and pmyc(H-P) are plasmids obtained by insertion into the plasmid pUC19 (Pharmacia) of the HindIII-KpnI region (about 1,200 bp) and the Hind III-PstI region (210 bp) of the human c-myc gene upstream region (cf. Reference 24), respectively. Since both have ARS activity, the core of ARS presumably occurs in the HindIII-PstI region of the human c-myc gene upstream region. This HindIII-PstI region determined by the dideoxy method has the following base sequence:

```
        1                    10                    20

        A A G C T T G T T T G G C C G T T T T A

        G G G T T T G T T G G A A T T T T T T

        T T C G T C T A T G T A C T T G T G A A

        T T A T T T C A C G T T T G C C A T T A

        C C G G T T C T C C A T A G G G T G A T

        G T T C A T T A G C A G T G G T G A T A

        G G T T A A T T T T C A C A T C T C T T

        A T G C G G T T G A A T A G T C A C C T

        C T G A A C C A A T T T T T C C T C C A

        G T A A C T C C T C T T T C T T C G G A

        C C T T C T G C A G

                             210
```

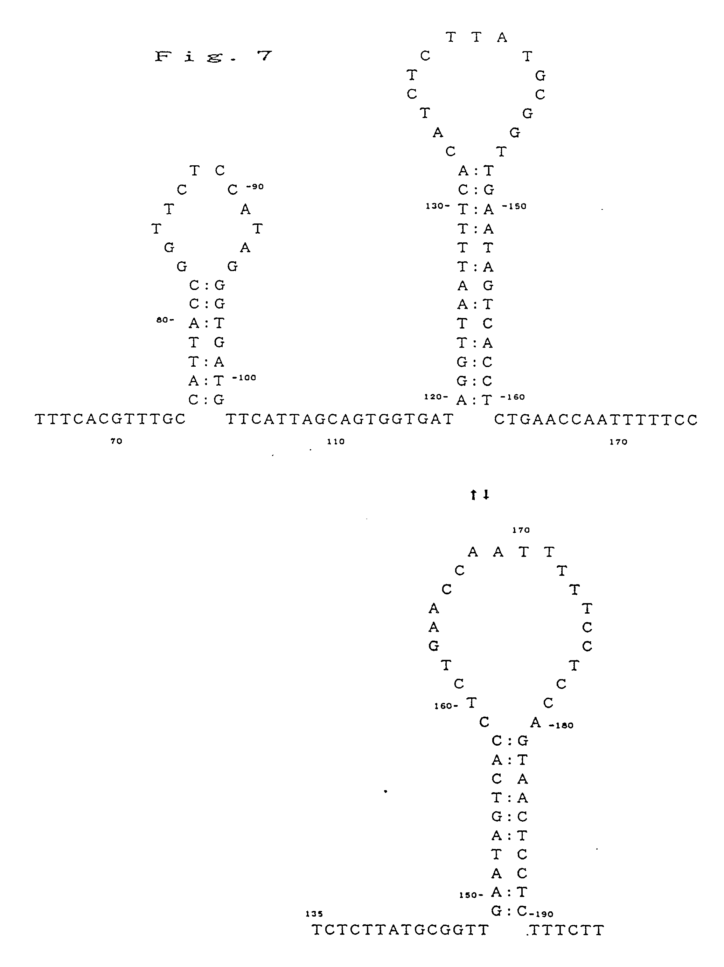

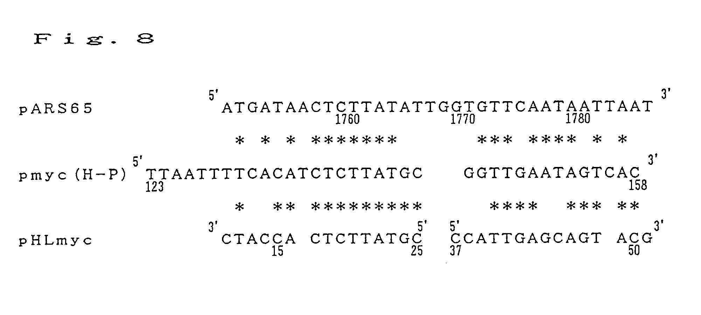

An estimated secondary structure as predicted from the above primary structure is shown in Fig. 7. The portions involving bases 76 to 101, bases 120 to 160 and bases 149 to 190, which respectively have a stem/loop structure, are thought to be important. Comparison of the ARS of pmyc(H-P) with a mouse ARS (pARS65) and a human ARS (pHLmyc) reveals a high degree of homology among them, as seen in Fig 8.

The HindIII-PstI fragment (210 bp) is excised from plasmid pmyc(H-P), c-myc protein is bound to this fragment and the base sequence of the region bound to c-myc protein is determined by the footprint analysis. As a result, the region shows the following 21 bp base sequence:

```
        T C T C T T A T G C G G T T G A A T A G T
```

This region corresponds to bases 135 to 155 of the HindIII-PstI fragment. In order to determine ARS activity of this base sequence, a DNA fragment having the above base sequence is chemically synthesized and inserted into a plasmid. Consequently, this plasmid is replicated in Hela cell in the episome state and therefore, it is considered that this 21 base sequence is particularly important for ARS activity. This ARS base sequence can be used for production of transgenic animal according to the present invention.

E. coli MV1184 harboring pmyc(H-P) has been deposited at the Fermentation Research Institute under the deposit number FERM BP-1932 in accordance with the Budapest Treaty.

pIMR-N is a human ARS-containing plasmid prepared by inserting into a plasmid a fragment capable of binding to a nuclear extract (N-myc protein) of human IMR32 cells as selected from HindIII-HindIII region fragments of the IMR32 cell-derived chromosomal DNA. pRJ-53 is likewise a human ARS-containing plasmid prepared by inserting a fragment capable of binding to a nuclear extract (p53 protein) of human Raji cells as selected from HindIII- HindIII region fragments of the Raji cell-derived chromosomal DNA.

A vector plasmid is constructed with an ARS, a promoter, the gene for a desired peptide, which is to be expressed, and so forth. The peptide gene should be disposed downstream from a promoter. The position and direction of an ARS in the vector plasmid are not particularly restrictred. It is convenient for construction of the vector plasmid to dispose an ARS upstream from a promoter or downstream from a terminator. A polyA signal for translation termination should preferably be disposed downstream from the peptide gene. For peptide production and secretion in a secretory organ or organs, a signal sequence-containing vector may be constructed. Any known signal peptide can be used.

The peptide to be produced is not limited to any particular species but may be selected optionally. Examples of such peptides, include proteins, glycoproteins and other medicinal substances, such as lymphokines (e.g. interferons such as interferon-$\gamma$ (Reference 26) and interferon-$\beta$ (Reference 27), and interleukins), insulin, growth hormone, human tissue plasminogen activator (Reference 28) and various enzymes.

For the expression of a foreign peptide gene in somatic cells of a transgenic animal, it is desirable that the expression be regulated by a protein occurring in somatic cells of the host. Therefore, the promoter to be used in the practice of the invention is preferably a promoter capable of regulating the mRNA tanscription level by means of a protein occurring in somatic cells of the host. Since different proteins are produced in different organs or tissues of the host, a suitable promoter should be selected depending on the organ or tissue in which the desired peptide is to be produced. Generally, the production of a desired peptide in an organ of the external secretion system is best suited for efficient collection of the desired peptide. Thus, for instance, where the desired peptide is to be produced in the mammary gland and to be recovered from milk, the casein promoter (References 29, 30, 31 and 32), whey acid protein (WAP) promoter (References 33, 34 and 35), $\alpha$-lactoglobulin promoter, $\beta$-lactoglobulin promoter, MMTV promoter or various regulatory regions (Reference 29), for instance, may be used. These promoters function in various animal species.

Likewise, where the desired peptide is to be produced in liver tissue, the hepatitis virus promoter or SAP promoter (Reference 36), for instance, may be used. For peptide production in the bone marrow, the immunoglobulin promoter may be used; for peptide production in the thymus, the T cell receptor promoter; for peptide production in chicken eggs, the ovalbumin promoter; and for peptide production in the brain, the $\gamma$-enolase promoter (Reference 37) may be used. In the practice of the invention, not only those promoters that regulate the expression of a foreign gene by binding with various cell-derived proteins but also those promoters that are regulated by an external stimulus may be used. For instance, a metallothionein promoter may be used so that the gene expression can occur in the liver or kidney where a heavy metal administered to the host accumulates, or a viral promoter may be used so that viral infection can lead to gene expression.

The desired peptide is preferably produced in the mammary gland and recovered from milk. For example, peptide production can be carried out by the following procedure.

Plasmid containing mouse WAP promoter, IGF-II gene derived from plasmid pIGF-II030 (E. coli K-12 MC 1061 IGF002 containing pIGF-II030 has been deposited at the Fermentation Reserch Institute under the accession number FERM BP-993), terminator and the above-described HindIII-PstI fragment (210 bp) as an ARS is constructed and dissolved in HEPES buffer. The plasmid-containing suspension is injected into mouse fertilized egg by means of micropipette and the egg is implanted into the uterus of the pseudopregnant host mouse which is previously subjected to surgical stimulus. The mouse is allowed to breed the transgenic mouse carrying the plasmid introduced into reproductive cells and somatic cells thereof. The thus-born transgenic mouse is grown to maturation and made to do coitus, parturition and lactation. The secreted milk is collected and the desired peptide, IGF, is recovered from milk.

All the gene manipulation procedures for the construction of the above-mentioned plasmid vectors can be performed in the conventional manner (cf. Reference 22, for instance).

The thus-produced production vector can be transferred to host animal embryonic cells by any known method, for example, the microinjection method (References 23 and 42). Namely, the production vector is dissolved in a buffer solution (e.g., HEPES buffer, phosphate buffer or physiological saline), this suspension is injected into the fertilized egg, preferably male pronuclei thereof, by means of a glass-made micropipette, and the egg is transplanted to the uterus of a pseudopregnant animal (the host animals become pseudopregnant by administering hormones (e.g., $PGF_{2\alpha}$, hCG, estradiol and LH) or, in case of small animals such as mice, physical stimulus).

The foreign gene introduced into an embryonic cell is distributed in all cells of the grown animal. The foreign gene is further present in reproductive cells, so that it is distributed in all somatic cells of the offspring of the animal. It is not necessary that the host animal to be used for foreign gene introduction thereinto should be of the same species as that from which the ARS is derived. In principle, transgenosis or gene transfer is possible in and among all animal species, inclusive of mammals, reptiles, birds, insects, etc. Examples of appropriate mammals are, for instance, mice, rats, rabbits, sheep, goats, cattle and horses. Among them, mice, goats and cattle are preferred.

With its growth, the thus-transformed transgenic animal produces the desired peptide upon endogenous or external stimulation. Large-quantity peptide production thus becomes possible.

The desired peptide thus produced may be recovered according to the method suitable for organs or tissues of the host animal in which the peptide is produced. For example, recovery of the desired peptide secreted in milk can be carried out as follows.

The desired peptide-containing milk is treated with acid such as hydrochloric acid to adjust a pH value of 4.2 to 4.6 (acid-precipitation) or treated with rennin in the presence of calcium ion (calcium-rennin precipitation) to form casein precipitate. When the desired peptide is present in supernatant (whey), it can be isolated and purified from whey in accordance with the methods conventionally used for isolation of proteins including gel filtration using a carrier such as agarose, cellulose, Sephadex; column chromatography using a carrier such as agarose, cellulose, Sephadex, DEAE-cellulose, Toyopearl; electrophoresis using polyacrylamide gel and the like; and high performance liquid chromatography. When the peptide is precipitated together with casein, its isolation is carried out by adding urea and 2-mercaptoethanol to milk and subjecting the mixture to the above-described isolation methods (References 38, 39 and 40).

In the case of oncogene introduction, namely when an oncogene product protein is the desired peptide, the transgenic animal may also be used in carcinogenesis tests or in anticancer agents screening by the method described in JP-A-61-81743 (the term "JP-A" as used herein means "an unexamined published Japanese patent application".) or U.S. Patent 4,736,866 corresponding thereto. Furthermore, a pathologic model animal may be created for use in drug screening, for instance, by introduction of a gene causative of a disease other than cancer. The invention includes such modes of practice as well.

The present invention is now illustrated in more detail by reference to the following example, but this example is not construed to limit the scope of the invention. Unless otherwise indicated below, all parts, percentages, ratios and the like are by weight.

## REFERENCE EXAMPLE

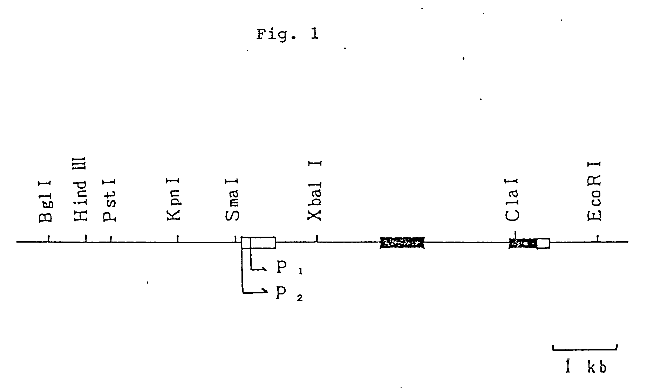

A human ARS-containing plasmid, pmyc(H-P), was construced using the human c-myc gene (Reference 24) and the plasmid pUC19 (Pharmacia). The human c-myc gene is a cellular tumor gene. While this gene is expressed also in normal cells, the level of its expression is very high in many tumor cells. Therefore, abnormal expression of c-myc is considered to be a cause for carcinogenesis. Fig. 1 shows the restriction enzyme cleavage map of the upstream region of this human c-myc gene. The present inventors have already revealed that an ARS is present in the HindIII-PstI region (210 bp) of this region (Reference 15).

The plasmid p-myc(H-P) was constructed by inserting the HindIII-PstI region excised from a human c-myc gene-containing plasmid (Reference 24) into the plasmid pUC19 (Pharmacia) in the HindIII-PstI region thereof and was amplified in dam$^+$ E. coli. The transformant E. coli MV1184 has been deposited at the Fermentation Research Institute under the deposit number FERM BP-1932 in accordance with the Budapest Treaty.

## EXAMPLE 1

### Transgenic Animal Production

The plasmid pmyc(H-P) obtained in Reference Example was dissolved in HEPES buffer and injected into the male pronuclei of fertilized mouse eggs (from $F_1$ of B6C3F1, C57/BL6 x C3H/Hen) by means of a glass-made micropipette and the eggs were transplanted to the uterus of a pseudopregnant mouse.

### Analysis of Introduced DNA

The entire tail DNA of one male and one female 3-week-old offspring ($F_0$) was extracted by the SDS-

7

proteinase K method (Reference 20). Each extracted DNA was subjected to agarose gel electrophoresis, followed by Southern blotting (Reference 24) using $^{32}$P-pmyc(H-P) as the probe. The introduced plasmid pmyc(H-P) was found completely as such in the form of extrachromosomal DNA.

The pmyc(H-P) introduced into the animal embryos was an amplification product obtained in dam$^+$ E. coli. In this E. coli strain, the adenine residue in the GATC site is methylated. Since, however, dam methylase is absent in animal cells, replicated pmyc(H-P), if newly replicated in the animal cells, must remain demethylated. Therefore, the tail DNA of each $F_0$ mouse was treated with DpnI, which cleaves the GATC site methylated at the adenine residue, and Mbol, which cleaves the GATC site demethylated at the adenine residue. This DNA showed DpnI resistance and Mbol susceptibility, indicating that pmyc(H-P) had been replicated in the mouse tail. Thus it was shown that pmyc(H-P) had been episomally replicated in the $F_0$ mouse tail without being taken up into host chromosomes.

Similarly, the tail DNA of each of eight offspring $F_1$ (two males and six females) obtained by mating $F_0$ males with $F_0$ females was examined in the same manner and it was found that pmyc(H-P) was present in the episome state. This means that pmyc(H-P) of the $F_0$ mice had been transmitted to their offspring via reproductive cells and amplified in a stable form. Furthermore, a total of 120 offspring mice obtained by $F_1$ x $F_1$ , $F_2$ x $F_2$ and $F_0$ x $F_1$ matings were examined. In all the mice, pmyc(H-P) was present extrachromosomally. Therefore, once introduced into individual mice as a plasmid, pmyc(H-P) can be transmitted very stably to the offspring.

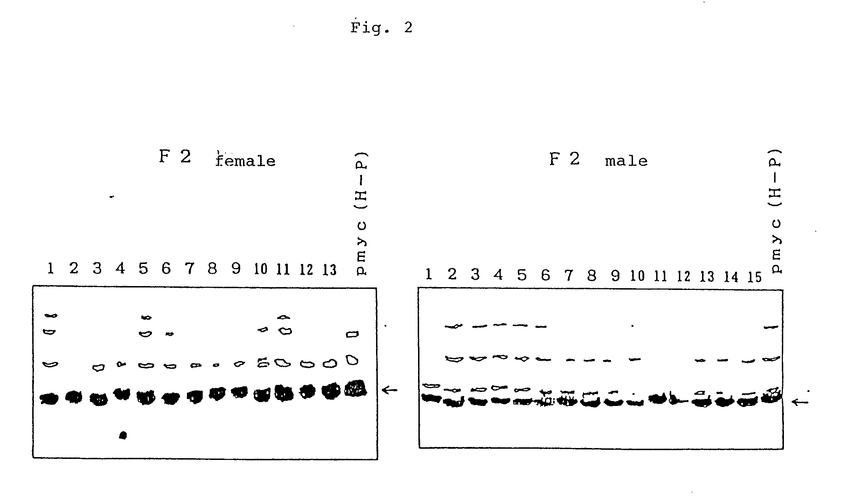

Fig. 2 shows Southern blot patterns of the tail DNA samples derived from $F_2$ mice (15 males and 13 females). These patterns show that pmyc(H-P) is present in the low-molecular-weight fraction indicated by the arrow but is absent in the high-molecular-weight fraction (at the top of each lane) corresponding to the chromosomal DNA and, in other words, that pmyc(H-P) is present as extrachromosomal DNA.

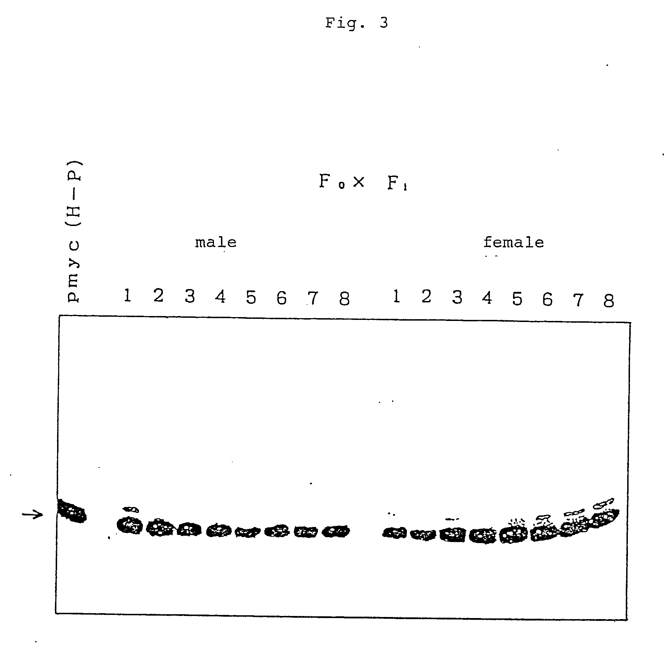

Fig. 3 shows Southern blot patterns of the tail DNA samples derived from mice (8 males and 8 females) obtained by $F_0$ (male) x $F_1$ (female) backcrossing. These patterns indicate that pmyc(H-P) can be transmitted in a stable manner to the offspring even after a plurality of generations.

## Intracorporeal Distribution of pmyc(H-P)

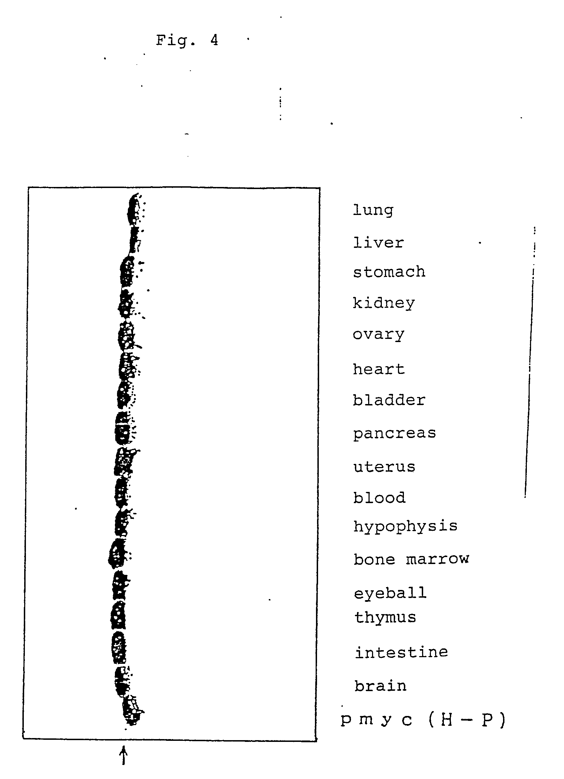

The intracorporeal distribution of pmyc(H-P) was examined by extracting the DNA of each $F_2$ mouse organ and subjecting the same to Southern blotting. As seen in Fig. 4, pmyc(H-P) was present stably in all the organs investigated. The copy number of pmyc(H-P) was 20 to 50 per cell. This means that the human ARS functions in every somatic cell of every transgenic animal.

While the data shown in Fig. 4 were obtained with female $F_2$ mice, male $F_2$ mice gave substantially the same results and pmyc(H-P) was stably present in the testis as well.

The above results establish that the human ARS-containing plasmid pmyc(H-P) can be maintained stably as an extrachrmosomal DNA in animal reproductive cells as well as in all somatic cells for 3 or more generations and that the plasmid has ARS activity and autonomously replicates in animal individuals as well. Thus, it has become possible to produce transgenic animals capable of producing an optionally seleted peptide in large quantities and with high efficiency by constructing an ARS-containing plasmid vector using an appropriate gene coding for the desired peptide and an appropriately selected promoter.

## EXAMPLE 2

## Identification of ARS

The ARS within the HindIII-PstI region of pmyc(H-P) was identified by the footprint method.

The foorprint method utilizes the fact that protein-bound DNA is blocked against reaction with DNase I. According to the method, a DNA treated with DNase I in the presence of a DNA-binding protein and a DNA treated with DNase I in the absence of a DNA-binding protein are subjected to high-resolution polyacrylamide electrophoresis suited for base sequence determination and the DNA band corresponding to the portion (protein binding site) protected from the attack of DNase I is observed in terms of the decrease in band intensity. The site protected from DNase I digestion can be determined accurately by performing the electrophoresis using, in parallel, the Maxam-Gilbert chemical reaction treatment products derived from the same DNA (Reference 43).

The HindIII-PstI region was excised from a human c-myc gene-containing plasmid (Reference 24), the c-myc protein (Reference 16) was allowed to bind thereto, and the c-myc protein bound DNA region was identified by the footprint method. Said DNA region was found to be composed of the following 21 bases:

1                                    10                              20 21

T C T C T T A T G C G G T T G A A T A G T

This DNA sequence corresponds to the bases 135-155 in the HindIII-PstI region (210 bp) of the human c-myc gene upstream region (cf. Fig. 7).

## EXAMPLE 3

## ARS activity of 21 bp

The above-identified 21-base DNA was synthesized by the conventional method.

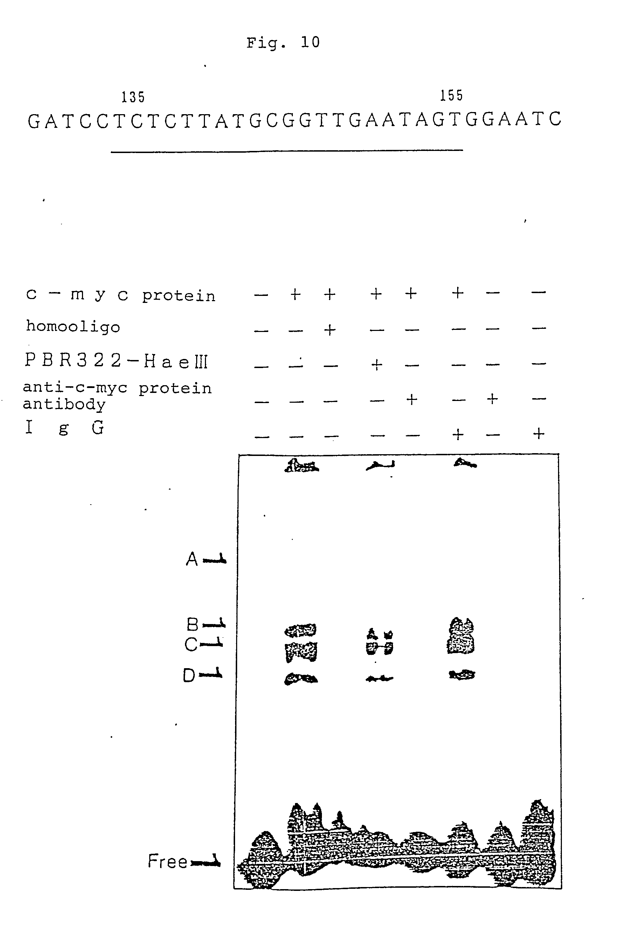

The 21-bp synthetic DNA was provided with BamHI sites (cf. the DNA sequence shown in the upper row in Fig. 10) and inserted into the plasmid pSVPCAT (which is a product of insertion, into the plasmid pUC18 (Pharmacia), of the CAT gene (Reference 44) and the SV40 promoter in adjacency to said CAT gene and has no enhancer region at the BamHI site upstream from the SV40 promoter region of the CAT gene. Thus was constructed a synthetic ARS-containing plasmid, pmyc-o-PCAT.

Human HeLa cells were transfected with this synthetic ARS-containing plasmid pmyc-o-PCAT and, after 40 hours of cultivation, a low-molecular-weight DNA fraction corresponding to the plasmid was extracted by the method of Hirt (Reference 45).

The extracted DNA was treated with EcoRI (for converting the circular plasmid to a linear DNA), then treated with DpnI or MboI, and subjected to agarose electrophoresis followed by Southern blotting using $^{32}$P-labeled pUC18 as a probe.

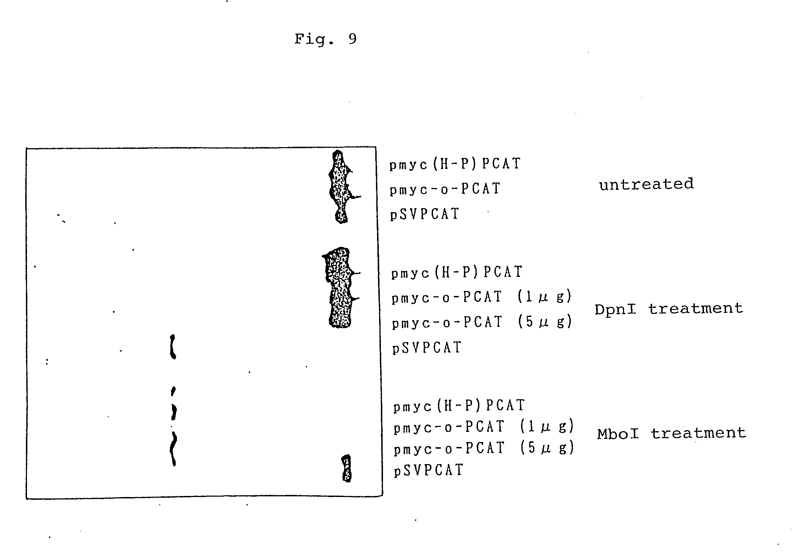

As shown in Fig. 9, the migration behavior of the pmyc-o-PCAT DNA was not influenced by DpnI treatment (DpnI resistance), but the DNA was cleaved upon MboI treatment (MboI susceptibility). It was thus shown that pmyc-o-PCAT had been replicated in HeLa cells or, in other words, that pmyc-o-PCAT had been replicated in an episomal state without being incorporated into any host chromosome. In this way, it was confirmed that the synthetic 21-bp DNA has an ARS activity.

In Fig. 9, there is also shown the result obtained with pmyc(H-P)PCAT constructed by modifying the HindIII-PstI region (210 bp) of the c-myc gene upstream region for converting both ends thereof to BamHI sites and inserting the resulting DNA into the plasmid pSVPCAT at the BamHI site, as in the construction of pmyc-o-PCAT. This plasmid, too, is resistant to DpnI and susceptible to MboI and can be replicated in HeLa cells.

The plasmid pSVPCAT which has no ARS sequence shows susceptibility to DpnI and resistance to MboI and shows no ARS activity (cf. the lane for pSVPCAT in Fig. 9).

Similar ARS assays performed with Raji cells, mouse L cells and other cells gave similar results, indicating that there is no species specificity regarding the replication potential of the synthetic ARS.

## EXAMPLE 4

## Binding of c-myc protein to synthetic ARS

The synthetic 21-bp ARS DNA was labeled with $^{32}$p and then mixed with a nuclear extract (producing the c-myc protein in large quantities) of human Raji cells, and gel shift assay was performed by subjecting the mixture to polyacrylamide electrophoresis.

As shown in Fig. 10, after mixing with the nuclear extract (c-myc protein), four bands, A, B, C, and D, corresponding to DNA-protein complexes and each showing a low extent of migration were observed. After dilution with the homooligo form (the synthetic ARS not yet labeled with $^{32}$P) or when the nuclear extract was treated with anti-c-myc antibody, these bands had disappeared. When DNA fragments, 20 to several hundred bp in size, prepared by HaeIII digestion of the plasmid pBR322 were simultaneously added, the bands showing a low extent of migration did not disappear. These results indicate that the synthetic ARS DNA and the c-myc protein are specifically bound to each other.

<div align="center">EXAMPLE 5</div>

Transcriptional control potential of synthetic ARS

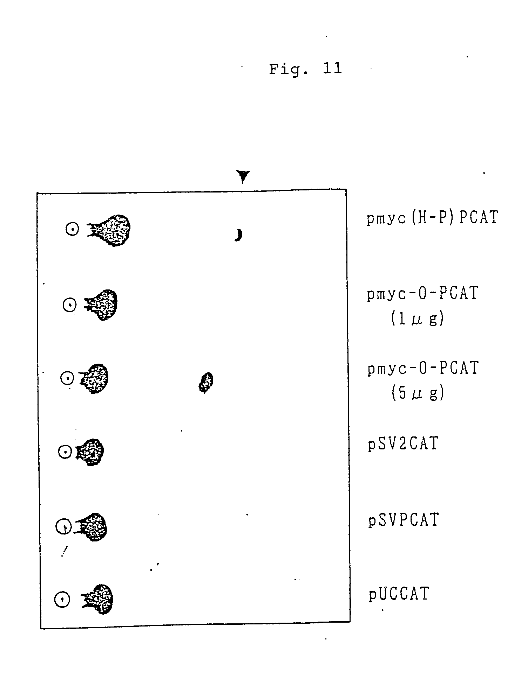

CAT assay was performed to see whether the synthetic 21-bp ARS DNA contains a transcriptional control region (Fig. 11).

CAT (chloramphenicol acetyltransferase) is an enzyme catalizing acetylation of the 1-position hydroxyl group or 3-position hydroxyl group or both. The transcriptional control function of a DNA sequence to be tested can be estimated by transforming cells with a vector containing the CAT gene and the DNA sequence to be tested and evaluating the activity of the translation product enzyme in terms of the acetylating potential of the cell extract.

HeLa cells were transformed with 1 or 5 $\mu$g/$10^6$ cells of pmyc-o-PCAT. After 2 days of culture, cells were collected, suspended in 200 $\mu$l of 0.25 M Tris-HCl, disrupted by three repetitions of freezing and then sonicated to give a cell extract. Chlorampheinicol was added to this cell extract and, after reaction, the reaction mixture was subjected to thin layer chromatography. A band (indicated by an arrow in Fig. 11) due to acetylated chloramphenicol was detected in the case of transfection with 5 $\mu$g of pmyc-o-PCAT. Similarly, acetylated chloramphenicol was detected also with the cell extract obtained after transfection with p-myc(H-P)PCAT.

On the other band, no acetylating potential was observed in the extract from cells transfected with pUCCAT (constructed by insertion of the CAT gene alone into the plasmid pUC19 at the HindIII site, having no SV40 promoter region) or with pPSVPCAT (constructed by inserting the SV40 promoter into pUCCAT). Any significant acetylating potential was observed with the original plasmid pSV2CAT constructed by introducing the SV40 enhancer into PSVPCAT at a site upstream from the promoter region) before insertion of the synthetic ARS, either.

These results indicate that the synthetic ARS has a potent enhancer activity which is similar to that of the HindIII-PstI region.

Similar assays were also performed using human Raji cells and mouse L cells and it was shown that the synthetic 21-bp ARS exhibits an enhancer activity in these cells as well.

The results mentioned above have led to the conclusion that the synthetic 21-bp ARS is not a mere replication origin but is equivalent or very close in function to an enhancer. This suggests that DNA-binding protein, such as the c-myc protein, are DAN replication initiating proteins and at the same enhnacer-binding preteins.

In Fig. 8, the DNA sequence of the 21-bp ARS is compared with those of other ARS species. The region that corresponds to the bases 135-155 in pmyc(H-P) is:

1756

A C T C T T A T A T T G G T G T T C A A T A A T

in the case of pARS65 or

C T C T A T G C and T T G A G C A G T A C

in the case of pHLmyc. Since these regions have a high level of homology, it is presumable that the DNA sequences of these homologous regions have an ARS activity as well and may be used as an autonomously replicating sequence (ARS), like the above-mentioned synthetic 21-bp DNA.

<div align="center">10</div>

As disclosed hereinabove, the invention provides transgenic animals, without species specificity problems or limitations on hosts, since, according to the invention, a plasmid vector containing a mammalian cell-derived autonomously replicating sequence (ARS) is used to transform animals. The plasmid vector introduced is maintained stably in the episome state and replicated extrachromosomally. Therefore, large-quantity experssion of the forein gene introduced become possible, hence the desired peptide can be produced in large amounts with good efficiency. In addition, the plasmid vector introduced will not be incorporated into host chromosomes, so that it will not cause any gene abnormalities in host animals unlike the situation in the prior art transgenic animals. Furthermore, once a generation of animals producing a desired peptide can be obtained, the offspring can be used also as production animals.

References

1. Wagner et al., Proc. Natl. Acad. Sci. USA, 78, 5016 (1981)
2. Stewart et al., Science, 217, 1046, (1982)
3. Constantini et al., Nature, 294, 92, (1981)
4. Lacy et al., Cell, 34, 343, (1983)
5. McKnight et al., Cell, 34, 335, (1983)
6. Brinster et al., Nature, 306, 332, (1983)
7. Palmiter et al., Nature, 300, 611, (1982)
8. Palmiter et al., Cell, 29, 701, (1982)
9. Palmiter et al., Science, 222, 809, (1983)
10. JP-A-61-81743 or US Pat. 4,736,866
11. JP-A-63-291 or EP 0264166-A1
12. Roth et al., Mol. Cell, Biol., 3, 1898-1908, (1983)
13. Ariga et al., Mol. Cell Biol., 7, 1-6 (1987)
14. Iguchi-Ariga et al. EMBO J., 6 2365-2371, (1987)
15. European Patent Application EP0254315A2
16. Science, 225, 718-720, (1984)
17. Nature, 296, 262-264, (1982)
18. Annu. Rev. Biochem., 52, 301-310 (1983)
19. Friend et al., Nature, 296, 262-264, (1982)
20. J. Viol., 34, 752-763 (1980)
21. Mol. Cell Biol., 3, 1958-1966 (1983)
22. Maniatis et al., Molecular Cloning:
A Laboratory Manual, Cold Spring Harbor Laboratory (1982)
23. Gordon et al. Proc. Natl. Acad. Sci. USA, 77, 7380, (1980)
24. Shibuya et al., Mol. Cell Biol., 5, 414-418 (1985)
25. Southern, J. Mol. Biol., 93, 503-517, (1975)
26. Nature, 295, 503 (1982)
27. Gene, 10, 11 (1980)
28. Nature, 301, 214 (1983)
29. J. Biochem., 101, 103 (1987)
30. J. Biochem., 99, 1639 (1986)
31. Nucleic Acds Research, 14, 1883 (1986)
32. Tanpakushitsu, Kakusan and Koso, 30(14), 1720 (1985)
33. Eur. J. Biochem., 125, 131 (1982)
34. Nucleic Acids Research, 12, 8685 (1984)
35. Proc. Natl. Acad. Sci. USA, 85, 5874 (1988)
36. J. Biochem., 100, 849 (1986)
37. Gene, 60, 103 (1986)
38. Biochemistry of Lactation, 77-94, edited by T.B. Mepham, Elsevier Science Publishers B.V., New York (1983)
39. Nippon Nogeikagaku Kaishi, 44(2), 89 (1970)
40. Eur. J. Biochem., 25, 505 (1972)
41. EMBO J., 7(10), 3135-3142 (1988)
42. Molecular Biology of the Gene, 4th edition, 814-817, J.D. Watoson et al., The Benjamin Cummings Publishing Company Inc. USA (1987)

43. Galas et al., Nucleic Acids Research, 5, 3157 (1978)

44. Alton et al., Nature, 282, 864 (1979)

45. Hirt, J. Mol. Biol., 26, 365-369 (1967)

While the invention has been described in detail and with reference to specific embodiments thereof, it will be apparent to one skilled in the art that various changes and modifications can be made therein without departing from the spirit and scope thereof.

## Claims

1. A transgenic animal carrying a plasmid introduced into reproductive cells and somatic cells thereof, said plasmid containing a mammalian cell-derived autonomously replicating sequence DNA, a promoter sequence and a peptide gene.

2. A transgenic animal as claimed in Claim 1, wherein said autonomously replicating sequence DNA is the HindIII-PstI region of the human c-myc gene upstream region.

3. A transgenic animal as claimed in Claims 1 to 3, wherein said autonomously replicating sequence DNA is

T C T C T T A T G C G G T T G A A T A G T

4. A transgenic animal as claimed in Claim 1, wherein the animal is mouse.

5. A method of producing a transgenic animal as claimed in Claim 1 which comprises injecting a plasmid containing a mammalian cell-derived autonomously replicating sequence DNA, a promoter sequence and a peptide gene into the male pronucleus of fertilized egg, transplaning the egg to the uterus of pseudopregnant host animal, and allowing the host animal to breed the transgenic animal.

6. A transgenic animal production method as claimed in Claim 5, wherein said autonomously replicating sequence DNA is the HindIII-PstI region of the human c-myc gene upstream region.

7. A transgenic animal production method as claimed in Claim 5, wherein said autonomously replicating sequence DNA is

T C T C T T A T G C G G T T G A A T A G T

8. A method of producing a peptide which comprises expressing a peptide gene in somatic cells of the transgenic animal of Claim 1.

9. A peptide production method as claimed in Claim 8, wherein said autonomously replicating sequence DNA is the HindIII-PstI region of the human c-myc gene upstream region.

10. A DNA having the following base sequence:

1          10         20

```
A A G C T T G T T T G G C C G T T T T A

G G G T T T G T T G G A A T T T T T T T

T T C G T C T A T G T A C T T G T G A A

T T A T T T C A C G T T T G C C A T T A

C C G G T T C T C C A T A G G G T G A T

G T T C A T T A G C A G T G G T G A T A

G G T T A A T T T T C A C A T C T C T T

A T G C G G T T G A A T A G T C A C C T

C T G A A C C A A T T T T T C C T C C A


G T A A C T C C T C T T T C T T C G G A

C C T T C T G C A G
```

210

11. A DNA having the base sequence:

```
T C T C T T A T G C G G T T G A A T A G T
```

Fig. 1

Fig. 2

Fig. 3

Fig. 4

lung

liver

stomach

kidney

ovary

heart

bladder

pancreas

uterus

blood

hypophysis

bone marrow

eyeball
thymus

intestine

brain

p m y c ( H − P )

Fig. 5

```
                GTA
             C      G
           A          C
          T            C
          A             T
           A           C
            G    G      G    T
             AT           G  T
             GC           CG
             TA           AT
             GC           TA
             CC           GC
             TA           AT
      12 —  AT      CG — 59
      GTATGATACAG   TGAG   GAAGAGACCATGCTCTGACACT
```

Fig. 6

```
                 TTG
              A       A
            C           G
             C     CAGTAC    G
               A   GTCATGT    T
                   CG
                   TA
                   GA
                   CG
                   TA
                   CG
            AGC        A
          T               C
            GCA         C
                   TA
                   AT
                   AG
                   GC
                   AT
       17 —        GC — 74
      GTATGATACAGATCGT   TGACACTGCACGACGTG
```

Fig. 7

```
                                        T   T   A
                                      C             T
                                    T                 G
                                    C                 C
                                      T             G
                                        A       G
                                          C   T
              T  C                          A : T
            C        C -90                  C : G
          T            A              130-  T : A  -150
        T                T                  T : A
          G              T                  T   T
          G            A                    T : A
            C : G                           A   G
            C : G                           A : T
      80-   A : T                           T   C
            T   G                           T : A
            T : A                           G : C
            A : T  -100                     G : C
            C : G                    120-   A : T  -160
   TTTCACGTTTGC      TTCATTAGCAGTGGTGAT      CTGAACCAATTTTTCC
        70                    110                      170
```

↑↓

```
                                170
                          A   A   T   T
                        C               T
                      C                   T
                    A                       T
                    A                       C
                    G                       C
                      T                   T
                        C               C
                  160- T               C
                        C       A -180
                        C : G
                        A : T
                        C   A
                        T : A
                        G : C
                        A : T
                        T   C
                        A   C
                  150-  A : T
                        G : C -190
            135
          TCTCTTATGCGGTT    .TTTCTT
```

Fig. 8

pARS65   5' ATGATAACTCTTATATTGGTGTTCAATAATTAAT 3'
         1760   1770   1780

      * * * *******   *** **** * *

pmyc (H-P) 5' TTAATTTTCACATCTCTTATGC  GGTTGAATAGTCAC 3'
      123             158

     * ** *********   **** *** **

pHLmyc   3' CTACCA CTCTTATGC 5' 5' CCATTGAGCAGT ACG 3'
        15    25 37      50

Fig. 8

Fig. 9

pmyc(H-P)PCAT
pmyc-o-PCAT          untreated
pSVPCAT

pmyc(H-P)PCAT
pmyc-o-PCAT (1μg)
pmyc-o-PCAT (5μg)    DpnI treatment
pSVPCAT

pmyc(H-P)PCAT
pmyc-o-PCAT (1μg)   MboI treatment
pmyc-o-PCAT (5μg)
pSVPCAT

EP 0 350 052 A2

Fig. 10

135                                      155

GATCCTCTCTTATGCGGTTGAATAGTGGAATC

_____

| | | | | | | | | |
|---|---|---|---|---|---|---|---|---|
| c − m y c protein | − | + | + | + | + | + | − | − |
| homooligo | − | − | + | − | − | − | − | − |
| P B R 3 2 2 − Ha e Ⅲ | − | − | − | + | − | − | − | − |
| anti-c-myc protein antibody | − | − | − | − | + | − | + | − |
| I g G | − | − | − | − | − | + | − | + |

A—

B—
C—

D—

Free—

EP 0 350 052 A2

Fig. 11

pmyc (H-P) PCAT

pmyc-O-PCAT
(1 μg)

pmyc-O-PCAT
(5 μg)

pSV2CAT

pSVPCAT

pUCCAT